# FASCICULE DE BREVET EUROPEEN

(11) **EP 2 730 243 B1**
(45) Date de publication et mention de la délivrance du brevet: **11.01.2017**
(21) Numéro de dépôt: 13191857.5
(22) Date de dépôt: 07.11.2013
(51) Int. Cl.: A61B 17/74

(54) **Dispositif d'ostéosynthèse pour le traitement des fractures du col de fémur**
Osteosynthesevorrichtung für die Behandlung von Knochenbrüchen des Oberschenkelhalses.
Osteosynthesis device for treating fractures of the femoral neck

(30) Priorité: 09.11.2012 FR 1260646
(43) Date de publication de la demande: 14.05.2014
(73) Titulaire: Pichon, Denis, 35510 Cesson-Sevigne (FR); Massin, Philippe, 75116 Paris (FR)
(72) Inventeur: Pichon, Denis, 35510 Cesson-Sevigne (FR); Massin, Philippe, 75116 Paris (FR)
(74) Mandataire: Regimbeau

(56) Documents cités:
- DE-A1-102007 029 090
- US-A1- 2001 034 523

## Description

La présente invention concerne un dispositif d'ostéosynthèse, utilisé dans la réparation des fractures du col du fémur.

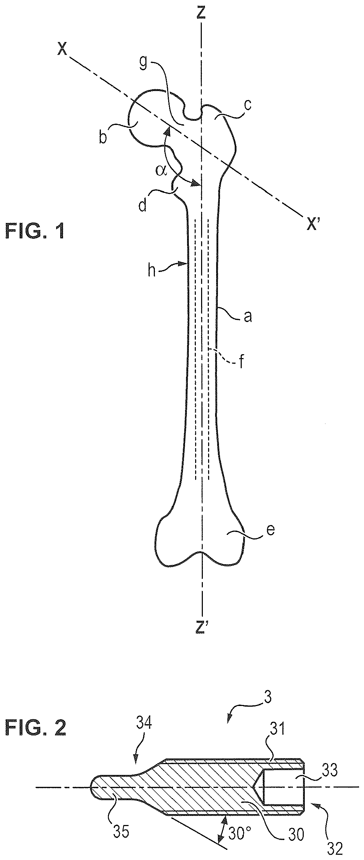

Pour mémoire, on rappellera ci-après les différentes parties constitutives du fémur, en se reportant à la figure 1 jointe.

Le fémur comprend une partie centrale allongée, dite "corps de fémur a" ou "diaphyse", à son extrémité proximale une tête de fémur b, légèrement en dessous le petit trochanter d, entre les deux et sur la face opposée de l'os, le grand trochanter c. Le col du fémur g relie la tête b au grand trochanter c. Enfin, le fémur présente à son extrémité distale un condyle e et son corps a est traversé par un canal médullaire f.

Les fractures du fémur surviennent souvent au niveau du col du fémur g ou dans la zone s'étendant entre le grand trochanter c et le petit trochanter d.

Afin de réduire la fracture, il est nécessaire de remettre l'os du fémur en position anatomique et de fixer les deux fragments osseux situés de part et d'autre de la fracture.

Les fractures du fémur surviennent souvent chez des personnes âgées, chez lesquelles il est souhaitable de réduire la durée d'une anesthésie et donc de l'opération chirurgicale.

Pour ce faire, et afin d'éviter la mise en place d'une prothèse de hanche, diverses techniques ont été mises au point.

Une première technique a consisté à visser une plaque sur la face extérieure de l'os, certaines vis coopérant avec la tête de fémur b, d'autres avec le corps a. Il existe toutefois un risque d'arrachement de cette plaque, lors des mouvements répétés de sollicitation de l'articulation, par exemple au moment du passage de la position assise à la position debout.

Afin de pallier cet inconvénient, on connaît également une autre technique dite "d'enclouage", qui consiste à insérer un élément allongé de section circulaire, connu de l'homme du métier sous la dénomination de "clou intramédullaire", à l'intérieur du canal médullaire f, selon son axe longitudinal Z-Z'. On insère ensuite à travers un orifice de passage ménagé dans ce clou, une vis, dénommée "vis céphalique". Cette insertion s'effectue depuis la face extérieure du grand trochanter c, selon l'axe X-X' du col du fémur. Ces deux éléments sont enfin assemblés l'un à l'autre de façon à garantir la bonne liaison entre la tête et le reste du fémur.

Le document EP 1 072 229 décrit un autre dispositif d'ostéosynthèse, dans lequel le montage est inversé puisque c'est le clou intramédullaire de section circulaire qui traverse un orifice de passage ménagé à cet effet dans la vis céphalique.

Les différents dispositifs précités présentent toutefois plusieurs inconvénients.

Tout d'abord, on sait que l'angle α (voir figure 1) entre l'axe X-X' du col du fémur g et l'axe Z-Z' du canal médullaire f varie d'un individu à un autre, et qu'il peut présenter des valeurs allant de 110° à 140° environ. Or, dans les dispositifs précités, l'angle entre le clou intramédullaire et la vis céphalique est fixe.

On connaît également d'après le document US 2001/0034523, un dispositif d'ostéosynthèse qui comprend cinq pièces, à savoir, un sabre fémoral, une vis céphalique munie d'un orifice transversal, un élément coulissant destiné à être inséré dans ladite vis céphalique et percé d'un orifice transversal de réception du sabre et d'un alésage axial fileté, une vis de blocage destinée à être insérée dans ledit alésage et une vis de compression destinée à être insérée axialement dans la vis céphalique.

Dans ce dispositif, le sabre fémoral est inséré dans l'orifice transversal de l'élément coulissant dont le diamètre correspond au jeu près à celui dudit sabre, et le sabre est maintenu dans l'élément coulissant avec la vis de blocage. L'angle entre le sabre et l'élément coulissant est donc fixe. Par ailleurs, la vis de compression permet uniquement de déplacer axialement l'élément coulissant à l'intérieur de la vis céphalique.

En conséquence, l'angle d'inclinaison entre le sabre fémoral et la vis céphalique est fixe. En outre ce dispositif est complexe et nécessite le stockage et la manipulation de cinq pièces.

Avec les techniques et dispositif précités, il est donc nécessaire d'avoir un grand nombre de clous intramédullaires ou de vis céphaliques dont les orifices de passage présentent des orientations angulaires différentes, de façon à pouvoir s'adapter au mieux à chaque patient.

En outre, la longueur du col du fémur peut également varier d'un individu à un autre. En conséquence, il est nécessaire d'avoir des jeux qui typiquement ont au moins dix à douze vis céphaliques et jusqu'à 50 clous intramédullaires, pour couvrir la gamme des différentes anatomies rencontrées chez les patients. Ce nombre de pièces important entraîne des problèmes de stockage. Par ailleurs, le chirurgien doit effectuer une sélection parmi un grand nombre de pièces, ce qui complique sa tâche.

De plus, les dispositifs de l'état de la technique présentent l'inconvénient que lorsque le clou intramédullaire et la vis céphalique sont assemblés, la tête de fémur b tend à se mettre préférentiellement en position de "varus", c'est-à-dire qu'elle tend à se rapprocher du petit trochanter d (l'angle α diminue), sans que les systèmes existants ne puissent favoriser une remise en "valgus" de la tête fémorale.

Enfin, la section du clou intramédullaire étant circulaire, il est nécessaire d'empêcher sa rotation axiale à l'intérieur du canal médullaire f, en le vissant à son extrémité inférieure, au travers du corps du fémur a. Or, il serait préférable que la reprise des efforts s'effectue plutôt sur la zone corticale interne du fémur, référencée h, située sous le petit trochanter d.

L'invention a pour but de résoudre ces inconvénients précités de l'état de la technique et de fournir un dispositif d'ostéosynthèse qui s'adapte à la morphologie d'un grand nombre de patients, notamment à la variation de l'angle entre l'axe X-X' du col du fémur et l'axe Z-Z' du canal médullaire et qui permette de limiter le nombre d'éléments intramédullaires fémoraux et de vis céphaliques de tailles différentes dans la gamme.

L'invention a également pour objectif de proposer un dispositif d'ostéosynthèse qui transmette les efforts appliqués sur la tête de fémur d'une manière plus anatomique que ceux connus de l'état de la technique, qui améliore la reprise des efforts exercés sur la tête de fémur, qui évite la mise en "*varus*" de la tête de fémur, et qui évite également la rotation de l'élément fémoral.

Enfin, l'invention a également pour objectif de fournir un dispositif d'ostéosynthèse qui soit simple à fabriquer et également simple à mettre en place par le chirurgien et à enlever si besoin est.

A cet effet, l'invention concerne un dispositif d'ostéosynthèse pour le traitement des fractures du col du fémur, comprenant un sabre fémoral de section transversale rectangulaire ou oblongue, destiné à être inséré dans le canal médullaire du fémur et une vis céphalique destinée à être insérée au moins en partie dans la tête et le col du fémur, ladite vis céphalique présentant un orifice transversal de réception dudit sabre fémoral.

Conformément à l'invention, ce dispositif comprend un élément de compression permettant d'accoupler ledit sabre fémoral et ladite vis céphalique, les deux parois transversales opposées, dites respectivement "distale" et "proximale", de l'orifice transversal sont inclinées dans le même sens par rapport à l'axe longitudinal (X1-X'1) de ladite vis céphalique, et la distance (D) entre l'arête dite "supérieure" de la paroi transversale distale située dans le plan de l'ouverture d'entrée dudit orifice transversal et la projection orthogonale de l'arête parallèle dite "inférieure" de la paroi transversale proximale dans ce même plan de l'ouverture d'entrée dudit orifice transversal est supérieure ou égale à la largeur (L) dudit sabre fémoral, le sabre fémoral, la vis céphalique et la vis de compression étant conçus de sorte que l'accouplement dudit sabre fémoral et de ladite vis céphalique est obtenu par l'élément de compression qui est lié à ladite vis céphalique et qui applique une pression sur l'une des faces latérales dudit sabre fémoral, de sorte que l'autre face latérale du sabre fémoral soit au contact de l'arête inférieure de la paroi transversale proximale de l'orifice transversal.

Grâce à ces caractéristiques de l'invention, l'angle entre le sabre fémoral et la vis céphalique est ajustable.

Selon d'autres caractéristiques avantageuses et non limitatives de l'invention, prises seules ou en combinaison :
- l'extrémité distale de la vis céphalique est dotée d'un alésage de réception dudit élément de compression, cet alésage débouchant dans ledit orifice transversal et s'étendant selon un axe distinct de l'axe longitudinal de ladite vis céphalique,
- l'axe de l'alésage est parallèle à l'axe longitudinal de ladite vis céphalique,
- l'axe de l'alésage et l'axe longitudinal de ladite vis céphalique sont sécants,
- ledit alésage est taraudé et l'élément de compression est une vis,
- l'angle entre l'axe longitudinal de ladite vis céphalique et le plan de la paroi transversale proximale de son orifice transversal est compris entre 30° et 70°,
- les angles de la section transversale rectangulaire du sabre fémoral sont arrondis, de préférence d'un rayon compris entre 0,2 mm et 4 mm,
- le sabre fémoral comprend une fente longitudinale à son extrémité distale, de sorte que celle-ci est partagée en deux branches,
- le sabre fémoral de section transversale rectangulaire présente sur au moins l'une de ses faces latérales une rainure longitudinale,
- l'élément de compression présente un nez dimensionné de façon à pouvoir être reçu dans ladite rainure longitudinale du sabre fémoral.

D'autres caractéristiques et avantages de l'invention apparaîtront de la description qui va maintenant en être faite, en référence aux dessins annexés, qui en représentent, à titre indicatif mais non limitatif, un mode de réalisation possible.

Sur ces dessins :
- la figure 1 est un schéma représentant un fémur,
- la figure 2 est une vue en coupe, à une échelle agrandie, de la vis de compression du dispositif d'ostéosynthèse de l'invention,
- la figure 3 est une vue en perspective de la vis céphalique du dispositif d'ostéosynthèse conforme à l'invention,
- les figures 4 et 5 sont des vues respectivement de dessus et en coupe de cette même vis céphalique,
- les figures 6 à 8 sont des vues respectivement de face, de côté, et en coupe selon la ligne VIII-VIII de la figure 6, du sabre fémoral du dispositif d'ostéosynthèse conforme à l'invention,
- la figure 9 est une vue de face de l'extrémité distale de la vis céphalique de la figure 4,
- les figures 10 et 11 sont des vues en coupe du dispositif d'ostéosynthèse complet, dans lesquelles le sabre fémoral et la vis céphalique sont orientés l'un par rapport à l'autre dans deux positions extrêmes.

Le dispositif d'ostéosynthèse conforme à l'invention comprend un sabre fémoral 1, une vis céphalique 2 et un élément de compression 3, par exemple une vis de compression et de préférence uniquement ces trois éléments. Le sabre fémoral 1 est destiné à être introduit à l'intérieur de la vis céphalique 2 et à être fixé à l'intérieur de celle-ci par l'élément de compression 3.

Chacun de ces éléments va maintenant être décrit plus en détail.

Dans la suite de la description et des revendications, les différents éléments du dispositif d'ostéosynthèse seront décrits en faisant référence à leurs extrémités dites respectivement "proximale" et "distale". Par convention, le terme "extrémité proximale" désigne l'extrémité de l'élément qui est la plus proche du coeur du patient, lorsque cet élément est mis en place sur le fémur dudit patient.

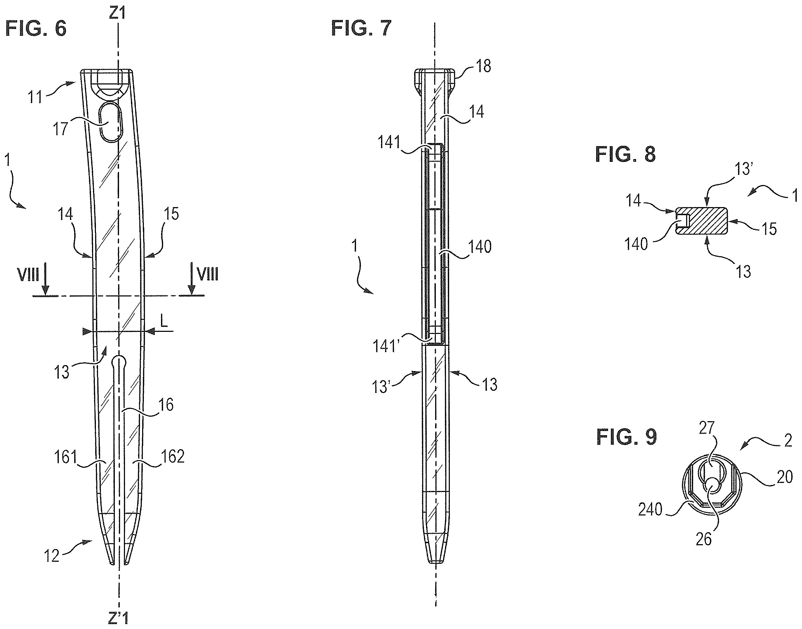

En se reportant aux figures 6 à 8, on peut voir que le sabre fémoral 1 est une pièce allongée (ou lame) ayant sensiblement la forme d'un parallélépipède rectangle dont la longueur est beaucoup plus importante que la largeur ou l'épaisseur.

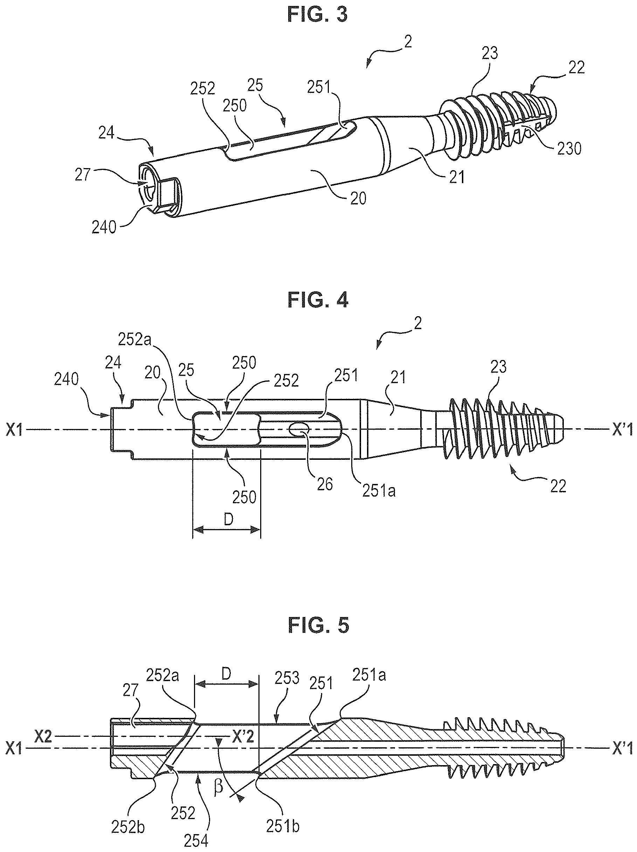

Le sabre 1 présente une extrémité proximale 11 et une extrémité distale 12. L'extrémité proximale 11 est en outre de préférence très légèrement incurvée (vers la gauche de la figure 5) par rapport à l'axe longitudinal médian Z1-Z'1 du sabre.

Le sabre 1 présente une face frontale 13 qui par convention est celle visible sur la figure 5, une face arrière opposée 13', et deux faces latérales plus étroites, à savoir une face latérale légèrement concave 14, et une face latérale légèrement convexe 15.

Comme on peut le voir sur la figure 8, le sabre 1 présente une section transversale rectangulaire, à angles arrondis, de préférence d'un faible rayon (0,2 mm à 4 mm) ou une section transversale oblongue.

De préférence, le sabre 1 présente en outre une fente 16 longitudinale qui s'étend sur environ les 2/5èmes de sa longueur totale, à partir de son extrémité distale 12.

Cette fente 16 délimite ainsi deux branches 161, 162 dont les extrémités sont avantageusement pointues.

De préférence, la fente 16 s'étend de la face frontale 13 jusqu'à la face arrière 13', comme représenté sur la figure 6. Elle pourrait également s'étendre de la face latérale concave 14 à la face latérale convexe 15.

Le sabre fémoral 1 est destiné à être inséré dans le canal médullaire f du fémur. La fente 16 donne une certaine élasticité aux deux branches 161 et 162 qui peuvent se rapprocher l'une de l'autre, ce qui combiné à la forme pointue de leurs extrémités favorise l'introduction du sabre dans ce canal.

En outre, de façon avantageuse, le sabre 1 présente sur sa face latérale concave 14 une rainure longitudinale 140 de faible profondeur (voir figure 8), et qui s'étend sensiblement sur les 2/5èmes de la longueur de la partie médiane du sabre, au-dessus de la fente 16.

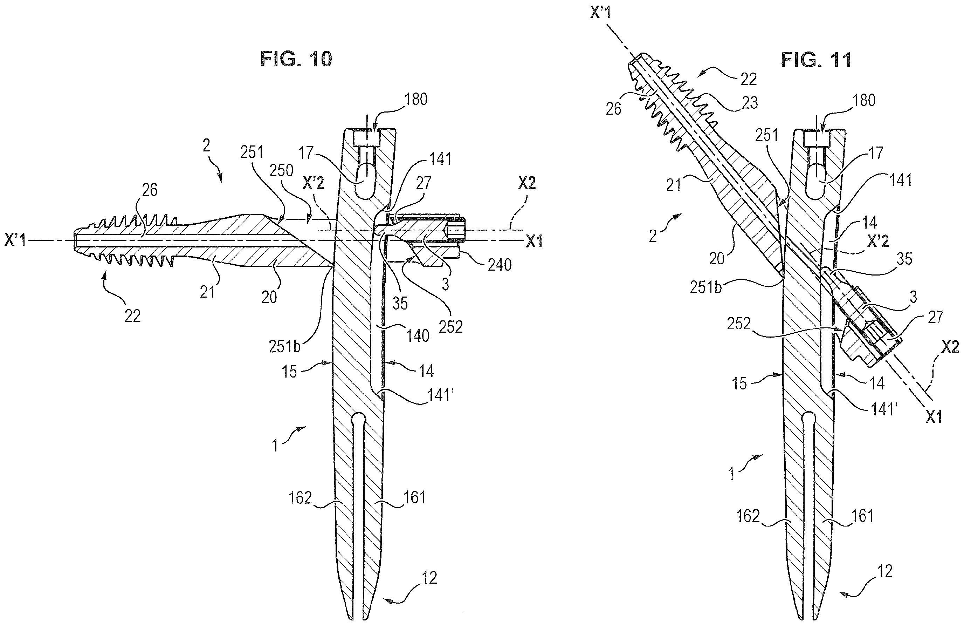

Comme cela apparaît mieux sur les figures 10 et 11, les deux extrémités 141 et 141' de la rainure 140 sont avantageusement inclinées depuis le fond de cette rainure en direction de la face latérale 14.

La rainure longitudinale 140 pourrait également être réalisée sur la face latérale 15, voire sur les deux faces 14 et 15.

Un trou oblong 17 est avantageusement ménagé à proximité de l'extrémité proximale 11 du sabre 1. Il traverse le sabre de part en part depuis sa face frontale 13 jusqu'à sa face arrière 13'.

Cet orifice permet au chirurgien d'y glisser un lien, afin de pouvoir exercer une traction sur le sabre 1 et retirer celui-ci du fémur si nécessaire.

L'extrémité proximale 11 proprement dite présente un renflement 18 ou "tête", faisant légèrement saillie sur les faces frontale 13 et arrière 13'. Un alésage 180 y est ménagé et il débouche à la partie supérieure du trou oblong 17, (voir figures 10 et 11).

Cet alésage 180 permet la fixation d'un outil de maintien du sabre 1 pendant son introduction dans le canal médullaire f ou son extraction hors de celui-ci.

A titre purement indicatif mais non limitatif, la longueur du sabre fémoral 1 est de l'ordre de 10 à 20 cm, la largeur L de sa face frontale 13 ou arrière 13' est d'environ 1 à 2 cm, et celle de ses faces latérales 14, 15 est d'environ 5 à 10 mm.

Comme on peut le voir sur les figures 3 à 5, la vis céphalique 2 présente la forme générale d'une tige d'axe longitudinal X1-X'1. Plus précisément, elle comprend un corps cylindrique 20, qui se prolonge à l'une de ses extrémités par une zone de diamètre décroissant 21, puis par l'extrémité proximale filetée 22.

De façon avantageuse, ce filetage est légèrement conique, c'est-à-dire que ses filets sont de plus faible hauteur à la pointe et progressivement de plus grande hauteur en direction de la zone 21.

De façon avantageuse également, le filetage 23 présente une zone d'interruption 230 du filetage, ce qui facilite l'introduction de la vis céphalique dans la tête du fémur. La vis 2 est ainsi "auto-foreuse".

Le corps 20 présente également une extrémité distale 24 dont la face 240 est de préférence perpendiculaire à l'axe longitudinal X1-X'1.

Un orifice transversal 25, de forme générale oblongue, est ménagé au travers du corps cylindrique 20. Il est destiné à recevoir le sabre fémoral 1.

Cet orifice 25 présente deux parois longitudinales parallèles 250, une paroi transversale proximale 251, et une paroi transversale distale 252.

Les deux parois transversales 251 et 252 sont inclinées par rapport à l'axe longitudinal X1-X'1 et dans le même sens, comme on peut le voir sur la figure 5.

Par ailleurs, l'orifice transversal 25 présente une ouverture d'entrée 253 par laquelle est introduit le sabre fémoral 1 et une ouverture de sortie 254 opposée. Ces ouvertures 253 et 254 s'étendent dans des plans parallèles (voir figure 5). Compte-tenu des différences d'inclinaison des parois transversales 251 et 252, l'ouverture d'entrée 253 est généralement plus longue que l'ouverture de sortie 254. La paroi transversale distale 252 présente une arête supérieure 252a et une arête inférieure 252b tandis que la paroi proximale 251 présente une arête supérieure 251 a et une arête inférieure 251 b.

Dans le mode de réalisation représenté sur les figures, les deux parois 251 et 252 ne sont pas parallèles. Elles pourraient toutefois l'être.

L'axe longitudinal X1-X'1 de la vis céphalique 2 forme un angle ß avec la paroi transversale proximale 251. Cet angle ß est compris entre 30° et 70°, de préférence 50° (L'angle ß est le complémentaire de l'angle entre l'axe X1-X'1 et la normale au plan de la paroi 251).

Par ailleurs, afin de permettre l'introduction du sabre fémoral 1, la distance D entre l'arête supérieure 252a de la paroi transversale distale 252 située dans le plan de l'ouverture d'entrée 253 dudit orifice transversal 25 et la projection orthogonale de l'arête parallèle inférieure 251 b de la paroi transversale proximale 251 dans ce même plan de l'ouverture d'entrée 253, est supérieure ou égale à la largeur L dudit sabre fémoral 1.

En outre, la vis céphalique 2 présente un alésage axial 26 qui s'étend depuis son extrémité proximale 22 jusqu'à l'orifice 250, à l'intérieur duquel il débouche au travers de la paroi 251. Cet alésage 26 s'étend le long de l'axe longitudinal X1-X'1 de la vis 2.

Enfin, la vis céphalique 2 présente au niveau de son extrémité distale 24, un alésage 27 qui débouche à l'une de ses extrémités sur la face 240 et à son autre extrémité au niveau de la face transversale distale 252.

Cet alésage 27 s'étend selon un axe longitudinal X2-X'2, qui peut être soit parallèle à l'axe X1-X'1, mais préférentiellement non coaxial avec celui-ci, (voir figures 5, 10 et 11), soit être sécant avec l'axe X1-X'1. Dans ce dernier cas, de préférence, l'alésage 27 est incliné de façon que son extrémité distale soit plus proche de l'axe X1-X'1 et son extrémité proximale en soit plus éloignée.

L'élément de compression 3 va maintenant être décrit. Il s'agit de préférence d'une vis de compression. Dans ce cas, l'alésage 27 est taraudé. L'élément 3 pourrait également être une clavette ou un levier articulé dès lors qu'il remplit la même fonction que la vis 3.

L'élément de compression 3 est formé d'une pièce unique qui est à la fois liée (c'est-à-dire fixée, par exemple vissée) avec la vis 2 et en contact avec le sabre 1 et qui garantit le blocage de l'un par rapport à l'autre.

Cette vis de compression présente un corps cylindrique 30 pourvu à sa périphérie d'un filetage 31 correspondant au taraudage de l'alésage 27 ménagé dans la vis céphalique 2.

Cette vis de compression 3 présente à son extrémité distale 32, une cavité 33, par exemple à pans coupés, afin de permettre l'introduction d'un tournevis de forme correspondante.

Enfin, son extrémité proximale 34 présente avantageusement un nez en saillie 35 à bout arrondi. Ses dimensions, et plus précisément son diamètre, sont tels qu'il peut pénétrer dans la rainure longitudinale 140 formée dans le sabre fémoral 1. En l'absence d'une telle rainure, le nez n'est pas nécessaire.

Le sabre fémoral 1, la vis céphalique 2 et la vis de compression 3 sont tous les trois réalisés dans des matériaux biocompatibles, par exemple alliages de titane, matériau composite ou acier inoxydable.

Après avoir réduit la fracture, c'est-à-dire après avoir rapproché la tête de fémur fracturée du reste du fémur et l'avoir replacée dans sa position anatomique d'origine, l'opérateur pratique un alésage à l'intérieur du canal médullaire f, puis une incision sensiblement selon l'axe X-X' du col du fémur, à partir du grand trochanter c et sans dépasser bien sûr une certaine distance du bord de la tête du fémur.

L'opérateur insère ensuite la vis céphalique 2 à l'aide d'une clé spécifique. Il fait pivoter la vis céphalique 2 jusqu'à ce que l'orifice transversal 25 soit aligné avec le perçage pratiqué dans le canal médullaire f. Il insère ensuite le sabre fémoral 1 à l'intérieur de l'orifice 25, comme représenté sur les figures 10 et 11.

Enfin, il visse la vis de compression 3, à l'intérieur de l'orifice taraudé 27 de la vis céphalique 2 jusqu'à ce que son extrémité proximale 34 (ou le nez 35) vienne au contact du sabre fémoral 1 (ou au fond de la fente 14 si celle-ci est présente) et que simultanément la face latérale opposée 15 du sabre vienne au contact de l'arête inférieure 251 b de la paroi transversale proximale 251, (voir figures 10 et 11). Ceci a pour effet de bloquer la position relative du sabre 1 par rapport à la vis céphalique 2.

L'opérateur ferme ensuite les incisions effectuées sur le patient.

L'inclinaison de la paroi proximale 251 par rapport à l'axe longitudinal X1-X'1, combinée au fait que la distance D est supérieure à la largeur L, permet d'avoir une plage importante d'inclinaisons possibles du sabre fémoral 1 par rapport à la vis céphalique 2, les deux positions extrêmes étant représentées respectivement sur les figures 10 et 11.

L'arête 251 b sert d'axe de pivotement du sabre fémoral 1 par rapport à la vis céphalique 2.

On voit donc qu'avec un seul couple de sabre fémoral 1 et vis céphalique 2, il est possible d'obtenir plusieurs orientations différentes et de s'adapter ainsi au mieux à la morphologie du patient et aux variations de l'angle α entre la tête de fémur b et le corps a. Le nombre total de sabres 1 et vis 2 dans la gamme s'en trouve ainsi réduit, il est seulement nécessaire de s'adapter à des grosseurs et longueurs de fémur différentes.

Par ailleurs, le fait que l'axe longitudinal X2-X'2 de l'orifice 27 soit excentré par rapport à l'axe longitudinal X1-X'1 de la vis céphalique 2 ou qu'il soit incliné de l'intérieur vers l'extérieur depuis la face 240 jusqu'à la face distale 252, est un moyen qui permet de modifier le point d'appui entre la vis de compression 3 et le sabre 1. Cette modification du point d'appui permet d'atteindre la position extrême optimisée représentée sur la figure 11.

La position excentrée ou inclinée de l'orifice 27 a également pour effet d'exercer une pression en un point plus haut du sabre 1, ce qui tend à ramener la vis céphalique 2 et conjointement la tête de fémur b en position de "valgus" (contraire à la tendance naturelle à la position de "varus" qui ne peut pas être rectifiée par les produits de l'état de la technique). L'angle α est augmenté.

La forme rectangulaire de la section transversale du sabre fémoral 1 empêche sa rotation autour de l'axe Z1-Z'1. De ce fait, il n'est plus nécessaire de le visser à sa base, au corps du fémur.

Enfin, la mise en place du dispositif d'ostéosynthèse est simple et le nombre de pièces le constituant est avantageusement limité au nombre de trois.

## Revendications

1. Dispositif d'ostéosynthèse pour le traitement des fractures du col du fémur, comprenant un sabre fémoral (1) de section transversale rectangulaire ou oblongue, destiné à être inséré dans le canal médullaire du fémur, une vis céphalique (2) destinée à être insérée au moins en partie dans la tête et le col du fémur, ladite vis céphalique (2) présentant un orifice transversal (25) de réception dudit sabre fémoral (1), et un élément de compression (3) permettant d'accoupler ledit sabre fémoral et ladite vis céphalique , ledit orifice transversal (25) comprenant deux parois transversales opposées, dites respectivement "distale" (252) et "proximale" (251), la distance (D) entre l'arête dite "supérieure" (252a) de la paroi transversale distale (252) située dans le plan de l'ouverture d'entrée (253) dudit orifice transversal (25) et la projection orthogonale de l'arête parallèle dite "inférieure" (251 b) de la paroi transversale proximale (251) dans ce même plan de l'ouverture d'entrée (253) dudit orifice transversal (25) étant supérieure ou égale à la largeur (L) dudit sabre fémoral (1), **caractérisé en ce que** lesdites parois distale (252) et proximale (251) de l'orifice transversal (25) sont inclinées dans le même sens par rapport à l'axe longitudinal (X1-X'1) de ladite vis céphalique (2), et **en ce que** l'élément de compression (3) est lié à ladite vis céphalique (2) et applique une pression sur l'une des faces latérales (14, 15) dudit sabre fémoral (1), de sorte que l'autre face latérale (15, 14) du sabre fémoral (1) soit au contact de l'arête inférieure (251 b) de la paroi transversale proximale (251) de l'orifice transversal (25).

2. Dispositif d'ostéosynthèse selon la revendication 1, **caractérisé en ce que** l'extrémité distale (24) de la vis céphalique (2) est dotée d'un alésage (27) de réception dudit élément de compression (3), cet alésage (27) débouchant dans ledit orifice transversal (25) et s'étendant selon un axe (X2-X'2) distinct de l'axe longitudinal (X1-X'1) de ladite vis céphalique (2).

3. Dispositif d'ostéosynthèse selon la revendication 2, **caractérisé en ce que** l'axe (X2-X'2) de l'alésage (27) est parallèle à l'axe longitudinal (X1-X'1) de ladite vis céphalique (2).

4. Dispositif d'ostéosynthèse selon la revendication 2, **caractérisé en ce que** l'axe (X2-X'2) de l'alésage (27) et l'axe longitudinal (X1-X'1) de ladite vis céphalique (2) sont sécants.

5. Dispositif d'ostéosynthèse selon l'une des revendications 2 à 4, **caractérisé en ce que** ledit alésage (27) est taraudé et **en ce que** l'élément de compression (3) est une vis.

6. Dispositif d'ostéosynthèse selon l'une des revendications précédentes, **caractérisé en ce que** l'angle (B) entre l'axe longitudinal (X1-X'1) de ladite vis céphalique (2) et le plan de la paroi transversale proximale (251) de son orifice transversal (25) est compris entre 30° et 70°.

7. Dispositif d'ostéosynthèse selon l'une des revendications précédentes, **caractérisé en ce que** les angles de la section transversale rectangulaire du sabre fémoral (1) sont arrondis, de préférence d'un rayon compris entre 0,2 mm et 4 mm.

8. Dispositif d'ostéosynthèse selon l'une des revendications précédentes, **caractérisé en ce que** le sabre fémoral (1) comprend une fente longitudinale (16) à son extrémité distale (12), de sorte que celle-ci est partagée en deux branches (161, 162).

9. Dispositif d'ostéosynthèse selon l'une des revendications précédentes, **caractérisé en ce que** le sabre fémoral (1) de section transversale rectangulaire présente sur au moins l'une de ses faces latérales (14, 15) une rainure longitudinale (140).

10. Dispositif d'ostéosynthèse selon la revendication 9, **caractérisé en ce que** l'élément de compression (3) présente un nez (35) dimensionné de façon à pouvoir être reçu dans ladite rainure longitudinale (140) du sabre fémoral (1).

11. Dispositif d'ostéosynthèse selon l'une des revendications précédentes, **caractérisé en ce qu'**il comprend exclusivement ledit sabre fémoral (1), ladite vis céphalique (2) et ledit élément de compression (3).

## Patentansprüche

1. Osteosynthesevorrichtung zur Behandlung von Oberschenkelhalsfrakturen, umfassend einen Femurstab (1) mit einem rechteckigen oder länglichen Querschnitt, der bestimmt ist, in den Knochenmarkskanal des Femurs eingesetzt zu sein, eine Zugschraube (2), die bestimmt ist, mindestens zum Teil in den Kopf und den Hals des Femurs eingesetzt zu sein, wobei die Zugschraube (2) eine transversale Empfangsöffnung (25) des Femurstabs (1) aufweist, und ein Kompressionselement (3), das erlaubt, den Femurstab und die Zugschraube zu koppeln, wobei die transversale Öffnung (25) zwei gegenüberliegende transversale Wände, jeweils als "distal" (252) und "proximal" (251) bezeichnet, umfasst, wobei der Abstand (D) zwischen der "oberen" Kante (252a) der distalen transversalen Wand (252) in der Ebene der Eingangsöffnung (253) der transversalen Öffnung (25) und der orthogonalen Projektion der "unteren" parallelen Kante (251b) der proximalen transversalen Wand (251) in derselben Ebene der Eingangsöffnung (253) der transversalen Öffnung (25) größer oder gleich der Breite (L) des Femurstabs (1) ist, **dadurch gekennzeichnet, dass** die distale (252) und proximale (251) Wand der transversalen Öffnung (25) in Bezug zur Längsachse (X1-X'1) der Zugschraube (2) in derselben Richtung geneigt sind, und dass das Kompressionselement (3) mit der Zugschraube (2) verbunden ist und einen Druck auf eine der Seitenflächen (14, 15) des Femurstabs (1) derart ausübt, dass die andere Seitenfläche (15, 14) des Femurstabs (1) im Kontakt mit der unteren Kante (251b) der proximalen transversalen Wand (251) der transversalen Öffnung (25) ist.

2. Osteosynthesevorrichtung nach Anspruch 1, **dadurch gekennzeichnet, dass** das distale Ende (24) der Zugschraube (2) mit einer Empfangsbohrung (27) des Kompressionselements (3) ausgestattet ist, wobei diese Bohrung (27) in die transversale Öffnung (25) ausmündet und sich gemäß einer Achse (X2-X'2) erstreckt, die sich von der Längsachse (X1-X'1) der Zugschraube (2) unterscheidet.

3. Osteosynthesevorrichtung nach Anspruch 2, **dadurch gekennzeichnet, dass** die Achse (X2-X'2) der Bohrung (27) parallel zur Längsachse (X1-X'1) der Zugschraube (2) ist.

4. Osteosynthesevorrichtung nach Anspruch 2, **dadurch gekennzeichnet, dass** die Achse (X2-X'2) der Bohrung (27) und die Längsachse (X1-X'1) der Zugschraube (2) schneidend sind.

5. Osteosynthesevorrichtung nach einem der Ansprüche 2 bis 4, **dadurch gekennzeichnet, dass** die Bohrung (27) gewindegeschnitten ist und dass das Kompressionselement (3) eine Schraube ist.

6. Osteosynthesevorrichtung nach einem der vorangehenden Ansprüche, **dadurch gekennzeichnet, dass** der Winkel (ß) zwischen der Längsachse (X1-X'1) der Zugschraube (2) und der Ebene der proximalen transversalen Wand (251) ihrer transversalen Öffnung (25) zwischen 30° und 70° inklusive beträgt.

7. Osteosynthesevorrichtung nach einem der vorangehenden Ansprüche, **dadurch gekennzeichnet, dass** die Ecken des transversalen rechteckigen Querschnitts des Femurstabs (1) abgerundet sind, vorzugsweise in einem Radius zwischen 0,2 mm und 4 mm inklusive.

8. Osteosynthesevorrichtung nach einem der vorangehenden Ansprüche, **dadurch gekennzeichnet, dass** der Femurstab (1) einen länglichen Schlitz (16) an seinem distalen Ende (12) umfasst, so dass dieses in zwei Schenkel (161, 162) geteilt ist.

9. Osteosynthesevorrichtung nach einem der vorangehenden Ansprüche, **dadurch gekennzeichnet, dass** der Femurstab (1) mit transversalem rechteckigen Querschnitt auf mindestens einer seiner Seitenfläche (14, 15) eine längliche Rille (140) aufweist.

10. Osteosynthesevorrichtung nach Anspruch 9, **dadurch gekennzeichnet, dass** das Kompressionselement (3) eine Nase (35) aufweist, die derart bemessen ist, dass sie in der länglichen Rille (140) des Femurstabs (1) aufnehmbar ist.

11. Osteosynthesevorrichtung nach einem der vorangehenden Ansprüche, **dadurch gekennzeichnet, dass** er ausschließlich den Femurstab (1), die Zugschraube (2) und das Kompressionselement (3) umfasst.

## Claims

1. An osteosynthesis device for the treatment of the femoral neck fractures, comprising a femoral saber (1) with a rectangular or oblong cross-section, intended to be inserted into the medullar channel of the femur, a cephalic screw (2) intended to be at least partly inserted into the head and the neck of the femur, said cephalic screw (2) having a transverse orifice (25) for receiving said femoral saber (1), and a compression element (3) giving the possibility of coupling said femoral saber and said cephalic screw, said transverse orifice (25) comprising two opposite transverse walls, respectively said to be "distal" (252) and "proximal" (251), the distance (D) between the so-called "upper" edge (252a) of the distal transverse wall (252) located in the plane of the inlet aperture (253) of said transverse orifice (25) and the orthogonal projection of the so-called "lower" parallel edge (251b) of the proximal transverse wall (251) in this same plane of the inlet aperture (253) of said transverse orifice (25) being greater than or equal to the width (L) of said femoral saber (1), **characterized in that** said distal (252) and proximal (251) walls of the transverse orifice (25) are tilted in the same direction with respect to the longitudinal axis (X1-X'1) of said cephalic screw (2), and **in that** the compression element (3) is bound to said cephalic screw (2) and applies pressure on one of the side faces (14, 15) of said femoral saber (1), so that the other side face (15, 14) of the femoral saber (1) is in contact with the lower edge (251b) of the proximal transverse wall (251) of the transverse orifice (25).

2. The osteosynthesis device according to claim 1, **characterized in that** the distal end (24) of the cephalic screw (2) is provided with a bore (27) for receiving said compression element (3), this bore (27) opening into said transverse orifice (25) and extending along an axis (X2-X'2) distinct from the longitudinal axis (X1-X'1) of said cephalic screw (2).

3. The osteosynthesis device according to claim 2, **characterized in that** the axis (X2-X'2) of the bore (27) is parallel to the longitudinal axis (X1-X'1) of said cephalic screw (2).

4. The osteosynthesis device according to claim 2, **characterized in that** the axis (X2-X'2) of the bore (27) and the longitudinal axis (X1-X'1) of said cephalic screw (2) are secant.

5. The osteosynthesis device according to one of claims 2 to 4, **characterized in that** said bore (27) is tapped and **in that** the compression element (3) is a screw.

6. The osteosynthesis device according to one of the preceding claims, **characterized in that** the angle (8) between the longitudinal axis (X1-X'1) of said cephalic screw (2) and the plane of the proximal transverse wall (251) of its transverse orifice (25) is comprised between 30° and 70°.

7. The osteosynthesis device according to one of the preceding claims, **characterized in that** the angles of the rectangular transverse section of the femoral saber (1) are rounded, preferably with a radius comprised between 0.2 mm and 4 mm.

8. The osteosynthesis device according to one of the preceding claims, **characterized in that** the femoral saber (1) comprises a longitudinal slot (16) at its distal end (12), so that the latter is divided into two branches (161, 162).

9. The osteosynthesis device according to one of the preceding claims, **characterized in that** the femoral saber (1) with a rectangular cross-section has on at least one of its side faces (14, 15) a longitudinal groove (140).

10. The osteosynthesis device according to claim 9, **characterized in that** the compression element (3) has a nose (35) dimensioned so as to be able to be received in said longitudinal groove (140) of the femoral saber (1).

11. The osteosynthesis device according to one of the preceding claims, **characterized in that** it exclusively comprises said femoral saber (1), said cephalic screw (2) and said compression element (3).
